(19) Europäisches Patentamt
European Patent Office
Office européen des brevets

(11) Publication number: **0 421 333 A1**

# (12) EUROPEAN PATENT APPLICATION

(21) Application number: **90118828.4**

(51) Int. Cl.[5]: **A61K 7/48**

(22) Date of filing: **01.10.90**

(30) Priority: **02.10.89 US 416177**

(43) Date of publication of application:
**10.04.91 Bulletin 91/15**

(84) Designated Contracting States:
**AT BE CH DE DK ES FR GB GR IT LI LU NL SE**

(71) Applicant: **BRISTOL-MYERS SQUIBB COMPANY**
**345 Park Avenue**
**New York, N.Y. 10154(US)**

(72) Inventor: **Parab, Prakash**
**267 Patton Place**
**Williamsville, New York 14221(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

(54) **Tretinoin emulsified cream formulations of improved stability.**

(57) An emulsified aqueous tretinoin cream formulation comprising a therapeutically active amount of tretinoin and from 0.01 to 5.0 percent by weight of an acrylic acid polymer as a stabilizer to prevent breaking of the emulsion in a pharmaceutically acceptable aqueous carrier is disclosed.

EP 0 421 333 A1

# TRETINOIN EMULSIFIED CREAM FORMULATIONS OF IMPROVED STABILITY

## Background of the Invention

This invention relates to stable tretinoin (also known as all-trans retinoic acid and vitamin A acid) cream formulations.

Acne vulgaris, the most common skin disease in the United States, affects some 17 million people. It is found most frequently in those aged 12 to 25 years old and accounts for about one-fifth of the practice of dermatology.

The acne lesions occur in sebaceous follicles, with maximum density existing on the face, chest and back. The characteristic lesion of acne is a comedone. Blackheads are open comedones, so colored due to melanin deposition. The follicle is filled with keratin, lipids and bacteria, and has a widely dilated orifice that allows its contents to escape.

Closed comedones, or whiteheads, are small and flesh colored and have microscopic openings that prevent the contents from escaping. Continued production of keratin and sebum may, however, lead to the rupture of the whitehead, releasing the contents into the dermis and subsequently initiating an inflammatory process. Inflammation close to the skin's surface results in the formation of papules and pustules, whereas in deeper sites the result is the formation of nodules and cysts. In severe cases, acne vulgaris will lead to permanent scarring, causing the patient mental pain and suffering.

A. M. Kligman (U.S. Patent 3,729,568) discloses the use of Vitamin A acid in the treatment of acne. This therapy was found to be markedly effective compared to prior methods, such as the use of peeling agents, hormone therapy (in females), antibacterial treatment and surgical skin planing.

More recently, A. M. Kligman (U.S. Patent 4,603,146) discloses the use of tretinoin to retard the aging of human facial skin. Normal aging and exposure to environmental traumas and sunlight lead to decreases in cellular turnover rates in the epidermis, a decrease in collagen fibers and the formation of abnormal elastic fibers in the dermis, causing the loss of elasticity and wrinkling of the skin. A leather-like consistency, mottling (hyperpigmentation), and various premalignant growths are also associated with the photoaging of the skin.

The elderly tend to have thin skin with decreased blood flow, accompanied by the skin's loss of sensory acuity and ability to heal. Tretinoin increases the proliferation of epidermal cells, thus thickening the epidermis, correcting atrophy and facilitating healing. Tretinoin also corrects the abnormal cell differentiation found in sun-damaged skin and reduces or stops hyperpigmentation. Furthermore, tretinoin stimulates dermal fibroblasts to produce new collagen and ground substance such as mucopolysaccharides, resulting in livelier, smoother, tighter skin possessing increased turgor. Finally, tretinoin stimulates blood flow and helps in the formation of new blood vessels in the skin. This in turn increases the following: skin temperature, clearance of irritants and toxins from the skin and sensitivity to pain and irritation.

In addition to acne treatment and treatment for photoaging, natural aging and other environmental traumas, other uses of Vitamin A acid have been reviewed by Thomas and Doyle (The Journal of the American Academy of Dermatology , Vol. 4, No. 5; May, 1981). These uses include the treatment of ichthyosis, psoriasis, acanthosis nigricans, lichen planus, molluscum contagiosum, reactive perforating collagenosis, melasma, geographical tongue, corneal epithelial peeling, Fox-Fordyce disease, cutaneous metastatic melanoma, keloids, hypertrophic scars, linear verrucous nevus, plantar warts, pseudofolliculitis, keratoacanthoma, solar keratosis of extremities, callosities and Darier's disease.

In the treatment of acne, A. M. Kligman (U.S. Patent 3,729,568) discloses solution formulations of tretinoin. The drug was dissolved in water miscible organic solvents such as ethyl alcohol, propylene glycol, polyethylene glycol and isopropyl alcohol. U.S. Patent 4,247,547 to Marks discloses an anhydrous tretinoin gel formulation containing organic solvents, such as ethyl alcohol, isopropyl alcohol and propylene glycol and a gelling agent such as an acidic carboxy polymer, e.g., Carbopol 934 and Carbopol 940, neutralized with an organic amine. This gel composition was demonstrated to be highly effective in treating acne and is capable of being stored without refrigeration as well.

The prior art has used organic solvents that are potential irritants and or stinging agents. Although propylene glycol has been used in topical formulations for quite some time, it has a history of causing irritation and sensitization (C. Huriez, "Allergy to Propylene Glycol", Rev. Franc. Allerg . 6:200, 1966; M. Hannuksela, V. Pivila and O.P. Salo, "Skin Reactions to Propylene Glycol", Contact Dermatitis, 1:112, 1975; S. Aagren-Jonsson and B. Magnusson, "Sensitization to Propantheline Bromide, Trichlorocarbanilide and Propylene Glycol in Anti-Perspirants", Contact Dermatitis , 2:79, 1976).

Furthermore, the glycols, for example propylene glycol and PEG-400, are hygroscopic. When used in

higher concentrations, they may absorb water from the skin, thus making it dry and irritated. The alcohols, such as ethyl alcohol and isopropyl alcohol, are also well known for causing stinging and drying effects when applied topically.

Tretinoin is known to be an irritant when applied topically (A. M. Kligman, U.S. Patent 3,729,568). Hence, it would be desirable to formulate tretinoin products devoid of stinging, irritating and sensitizing agents. One solution is a cream formulation in which either oil is dispersed in water or water is dispersed in oil. Emollients or lubricating agents present in the cream will be beneficial because they may reduce the erythema, itching and stinging associated with tretinoin. Also, water will hydrate the skin, reducing the irritation caused by dry skin.

A cosmetic cream formulation having Vitamin A acid for the regulation of cornification in human skin is disclosed in British Patent 906,000. No mention is made of its use for the treatment of acne.

British Patent 1,466,062 discloses both cream and solution formulations of tretinoin that bring marked cosmetic improvement to the skin and also condition the nails and hair.

Retin-A, a cream formulation containing tretinoin, has been produced for the commercial market. This formulation is disclosed in Physicians' Desk Reference , 43d Edition, page 1517 (1989). The formulation is described as containing tretinoin in either of two strengths, 0.1% or 0.05% by weight, in a hydrophilic cream vehicle of stearic acid, stearyl alcohol, xanthan gum, sorbic acid, butylated hydroxytoluene, and purified water.

U.S. Patent 3,906,108 to Felty discloses a stabilized cream emulsion of tretinoin, capable of being stored without refrigeration for long periods of time without losing therapeutic effectiveness and while maintaining the uniformity and stability of the cream, containing xanthan gum as the stabilizer to prevent breaking of emulsion.

The United States Pharmacopeia (USP) XXI, page 1075, specifies that during its shelf life, the marketed tretinoin cream should contain not less than 90% and not more than 130% of the labelled amount of tretinoin. The 30% overage allowed by the USP is due to the instability of tretinoin in cream formations. Tretinoin may degrade by free radical meditated oxidation, photochemical reactions, heat mediated reactions, and isomerization. Furthermore, tretinoin may react with the excipients in the formulation, resulting in degradation by-products. These by-products may be irritating, sensitizing or phototoxic, or may have unknown side effects. Hence, it would be preferable to formulate a tretinoin cream product for the market in which degradation is minimal. This would result in a reduction of by-products, and therefore side effects, during the product shelf-life.

A booklet published by B. F. Goodrich entitled "Carbopol® Water Soluble Resins" discloses that Carbopol® resins are acrylic acid polymers, and are useful as thickeners in topical pharmaceutical applications.

## Summary of the Invention

Emulsified aqueous cream formulations of tretinoin, which are capable of being stored without refrigeration for long periods of time without losing therapeutic effectiveness and while maintaining physical and chemical stability, have been discovered. Physical stability refers to the stability of the emulsion, i.e., whether or not it breaks upon storage, whereas chemical stability refers to the amount of tretinoin remaining in the emulsion after storage. The emulsified aqueous tretinoin cream formulation of this invention comprises a therapeutically active amount of tretinoin and from 0.01 to 5.00 percent by weight of an acrylic acid polymer as a stabilizer to prevent breaking of the emulsion in a pharmaceutically acceptable aqueous carrier. The acrylic acid polymer contains repeating units having the structure:

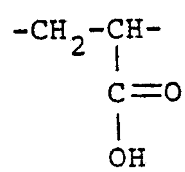

cross-linked with an allyl ether of pentaerythritol or an allyl ether of sucrose.

## Detailed Description of the Invention

The acrylic acid polymer used in the practice of this invention may be a Carbopol® resin, also known as carbomer. The acrylic acid polymer preferably has a molecular weight of from about 450,000 to 4,000,000 and, in the most preferred embodiment, has an approximate molecular weight of 3,000,000.

In another preferred embodiment, the polymer contains repeating units of acrylic acid and an alkyl methacrylate having the structure

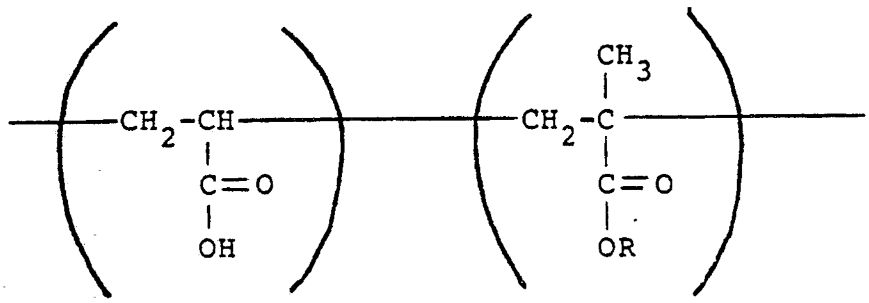

wherein R is an alkyl group or an alkenyl group containing from 10 to 30 carbon atoms cross linked with an allyl ether of pentaerythritol or an allyl ether of sucrose.

The pharmaceutically acceptable carrier for the tretinoin and acrylic acid polymer may include emulsifiers, thickening agents, anti-oxidants, emollients, preservatives, chelating agents, neutralizing agents for the acrylic acid polymer, and water. It is preferred that the formulation contain from about 0.005 to 0.5 percent by weight of tretinoin.

Non-ionic and anionic emulsifiers can be used in the composition of the present invention. However, non-ionic emulsifiers are preferred, specifically polyoxyethylene-2-stearyl ether, polyethylene glycol-23-lauryl ether, polyoxyethylene-21-stearyl ether, polyoxyethylene-4-lauryl ether, polyethylene glycol-6-ceteryl ether, polyethylene glycol-25 ceteryl ether, sorbitan monostearate and polyoxyethylene-20-sorbitan monostearate, to name a few. In the composition of this invention, it is preferred to have from about 1% up to about 15% by weight of non-ionic emulsifiers.

A solid to semi-solid hydrophobic material can be used in this formulation which includes fatty alcohols having 12 to 20 carbon atoms, for example cetyl alcohol, stearyl alcohol and ceto-stearyl alcohol. Also, pharmaceutical grades of white wax, sperm wax, bees wax, cocoa butter, hydrocarbon waxes, petrolatum, and fatty acids having 12 to 20 carbon atoms, for example stearic acid and palmitic acid, and finally, esters of fatty acids, such as glyceryl monostearate and cetyl palmitate, can be included. It is preferred to have from 1% up to about 25% by weight of solid to semi-solid hydrophobic material in the composition of the invention.

A liquid hydrophobic material can be used which includes fatty acid esters, where the fatty acid moiety has from about 6 to 20 carbon atoms, such as dibutyl adipate, diisopropyl adipate, and other esters of adipic acid, hexyl laurate, isopropyl myristate, propylene glycol dipelargonate and hydrocarbons such as mineral oil and squalane. Preferably from about 1% to about 50% by weight of the composition of this invention should be present as liquid hydrophobic material.

Suitable preservatives can be used for the formulation which include diazolidinyl urea, methyl isothiazolinone and methyl chloroisothiazolinone, benzyl alcohol, DMDM hydantoin (1,3-dimethylol-5,5-dimethyl hydantoin), methyl paraben, and propyl paraben, to name a few. It is preferred that from 0.01% up to 3.0% by weight of preservative be included in the formulation of the present invention.

Antioxidants can be used for the composition which include butylated hydroxytoluene, butylated hydroxyanisole, propyl gallate, and dl-tocopherol, to name a few. A preferred range for antioxidants in the composition of this invention is from 0.01% up to 5.0% by weight.

Materials such as chelating agents, humectants, sunscreens, moisturizers, dyes and perfumes may be included in the composition of the invention with discretion. Common examples of such additives are glycerin, sorbitol, citric acid, sucrose cocoate, methyl gluceth P-20, ethylene diamine tetracetic acid and its metal salts, octyl methoxy cinnamate and oxybenzone.

In combination with the carbomer, other thickening agents such as magnesium aluminium silicate may be used, but this is not essential to the invention.

The formulation of this invention may be used in the treatment of acne or other dermatological conditions where tretinoin is indicated or to retard the effects of aging of the skin. The formulations are applied topically to the area to be treated at regular intervals, as needed. The duration of the treatment will depend upon the nature and severity of the condition to be treated as well as the frequency of application of the formulation.

The following two tretinoin cream formulations were prepared which are both physically and chemically stable for long periods of time.

| | Parts by Weight | | |
|---|---|---|---|
| Ingredient | Example 1 | Example 2 | Function |
| Tretinoin | 0.1 | 0.05 | Active Ingredient |
| Petrolatum | 6.0 | 6.0 | Moisturizer |
| Dimethicone 200 | 1.0 | 1.0 | Moisturizer |
| Cetyl alcohol | 1.6 | 1.6 | Emollient/thickening agent |
| Polyoxyethylene-23-lauryl ether | 0.5 | 0.5 | Nonionic emulsifier |
| Butylated hydroxytoluene | 0.05 | 0.05 | Antioxidant |
| Butylated hydroxyanisole | 0.05 | 0.05 | Antioxidant |
| Polyoxyethylene-2-stearyl ether | 2.5 | 2.5 | Nonionic emulsifier |
| Cocoa butter | 8.0 | 8.0 | Emollient |
| Dibutyl adipate | 10.0 | 10.0 | Solvent for tretinoin/emollient |
| Glycerin | 5.0 | 5.0 | Moisturizer |
| Carbomer 934 | 0.25 | 0.25 | Thickener |
| Magnesium aluminum silicate | 0.30 | 0.30 | Thickener |
| A mixture of methylchloroisothiazolinone and methylisothiazolinone (Kathon CG) | 0.05 | 0.05 | Preservative |
| Diazolidinyl urea | 0.20 | 0.20 | Preservative |
| Sodium hydroxide | 0.07 | 0.07 | Neutralizing agent for carbomer |
| Disodium ethylenediaminetetraacetic acid | 0.05 | 0.05 | Chelating agent |
| Purified Water, USP | 64.28 | 64.33 | Vehicle |

The method for processing each of these examples is set forth below:

Method of Processing

1. In a suitable size premix vessel, warm the dibutyl adipate to 45-50° C. With rapid mixing, add tretinoin and continue the mixing until uniform, while maintaining the temperature between 45-50° C.
2. In a separate premix vessel, warm the following to 65° C: petrolatum, dimethicone 200, cetyl alcohol, polyoxyethylene-23-lauryl ether, butylated hydroxytoluene, butylated hydroxyanisole, polyoxyethylene-2-stearyl ether and cocoa butter. Mix until uniform, maintaining the temperature between 60-65° C.
3. Add 95% of the water to the main mix vessel. While rapidly mixing, add the glycerin, magnesium aluminum silicate and carbomer 934. Mix until the magnesium aluminum silicate and carbomer 934 are well dispersed. Then with continued rapid mixing, warm and maintain the temperature at 60-65° C.
4. In a separate premix vessel, dissolve the sodium hydroxide in 1% of the water. Add this solution to step 3 water phase contents and continue mixing while maintaining temperature to 60-65° C.
5. With rapid mixing, add the step 2 oil phase contents to step 3 water phase contents and mix to form an emulsion while maintaining the temperature at 60-65° C. Then add step 1 tretinoin solution and continue rapid mixing while cooling to between 40-45° C.
6. In a small premix vessel, disperse the disodium ethylenediaminetetraacetic acid in 1% of water. Add this suspension to the step 5 cream and mix.
7. In a small premix vessel, dissolve diazolidinyl urea and Kathon CG in the remaining 3% of water. Add this solution to the step 5 cream and mix moderately while cooling to room temperature.

In the formulations of Examples 1 and 2, "dimethicone 200" is dimethyl polysiloxane and "carbomer 934" is an acrylic acid polymer which contains repeating units having the structure

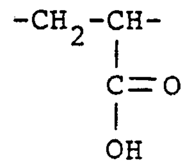

which is cross-linked with an allyl ether of sucrose and has an approximate molecular weight of 3,000,000.

Table 1 sets forth the concentrations of tretinoin in the cream formulations of Examples 1 and 2 at room temperature (RT) and at 40 °C as determined at different time intervals. It will be appreciated that experimental error gave rise to those instances wherein more than 100% of tretinoin was found to be in the formulation after storage.

In Table 1 "% (w/w)" means the percent by weight of tretinoin based upon the weight of the total composition; and "% Initial" means the percent of tretinoin remaining after storage for the designated times and temperatures based upon the initial amount of tretinoin in the formulation. Table 1 illustrates that the chemical stability of the emulsified tretinoin cream formulations of this invention is very high.

TABLE 1

| Concentrations of tretinoin in cream formulation at room temperature (RT) and 40 °C as determined at different time intervals. | | | | |
|---|---|---|---|---|
| Time Temperature | Example 1 | | Example 2 | |
| | % (w/w) | % Initial | % (w/w) | % Initial |
| Initial | 0.100 | | 0.0536 | |
| 4 Week 40 °C | 0.099 | (99.00) | 0.052 | (97.0) |
| 8 Week 40 °C | 0.0984 | (98.40) | 0.0516 | (96.2) |
| 12 Week 40 °C | 0.096 | (96.00) | 0.0511 | (95.3) |
| 26 Week 40 °C | 0.096 | (96.00) | Sample not evaluated | |
| 12 Week RT | 0.100 | (100.00) | 0.0530 | (98.8) |
| 26 Week RT | 0.102 | (102.00) | 0.053 | (98.8) |
| 39 Week RT . | Sample not evaluated | | 0.0523 | (97.5) |
| 1 Year RT | 0.101 | (101.00) | 0.0522 | (97.3) |

The physical stability of the emulsified tretinoin cream formulation of this invention is also very high. Thus, these formulations show no separation when stored at 40 °C (104 °F) for 26 weeks.

Examples 3, 4, 5 and 6 illustrate the criticality of using carbomer as a stabilizer to prevent breaking of the emulsion.

| Ingredient | Example 3 %w/w | Example 4 %w/w | Example 5 %w/w | Example 6 %w/w |
|---|---|---|---|---|
| Tretinoin | 0.1 | 0.1 | 0.1 | 0.1 |
| Petrolatum | 6.0 | 6.0 | 6.0 | 6.0 |
| Dimethicone 200 | 1.0 | 1.0 | 1.0 | 1.0 |
| Cetyl alcohol | 1.6 | 1.6 | 1.6 | 1.6 |
| Polyoxyethylene-23-lauryl ether | 0.5 | 0.5 | 0.5 | 0.5 |
| Butylated hydroxytoluene | 0.05 | 0.05 | 0.05 | 0.05 |
| Butylated hydroxyanisole | 0.05 | 0.05 | 0.05 | 0.05 |
| Polyoxyethylene-2-stearyl ether | 2.5 | 2.5 | 2.5 | 2.5 |
| Cocoa butter | 8.0 | 8.0 | 8.0 | 8.0 |
| Dibutyl adipate | 10.0 | 10.0 | 10.0 | 10.0 |
| Glycerin | 5.0 | 5.0 | 5.0 | 5.0 |
| Carbomer 934 | 0.00 | 0.25 | 0.00 | 0.00 |
| Magnesium aluminum silicate | 0.30 | 0.00 | 0.00 | 1.00 |
| A mixture of methylchloroisothiazolinone and methylisothiazolinone (Kathon CG) | 0.05 | 0.05 | 0.05 | 0.05 |
| Diazolidinyl urea | 0.20 | 0.20 | 0.20 | 0.20 |
| Sodium hydroxide | 0.00 | 0.07 | 0.00 | 0.00 |
| Disodium ethylenediaminetetraacetic acid | 0.05 | 0.05 | 0.05 | 0.05 |
| Purified Wqter, USP | 64.60 | 64.58 | 64.90 | 63.90 |

The physical stability of these examples at 45° C (113° F) is given in Table 2.

Table 2

| | Example | | | |
|---|---|---|---|---|
| Time | 3 | 4 | 5 | 6 |
| 1 week | S | N | S | S |
| 2 week | - | N | - | - |
| 3 week | - | N | - | - |
| 4 week | - | N | - | - |
| 5 week | - | N | - | - |
| 6 week | - | N | - | - |
| 7 week | - | N | - | - |
| 8 week | - | N | - | - |
| 9 week | - | N | - | - |
| 10 week | - | N | - | - |

In the above table, "N" means no separation and "S" means that the oil and water phases have separated. As a rule, an emulsion showing no separation when stored at 45° C (113° F) for eight weeks should be stable for a minimum of two years when stored at room temperature.

The data for samples stored at high temperature indicates that carbomer (Example 4) effectively stabilizes the cream, whereas sample formulations stored at high temperatures without carbomer (Examples 3 and 6 - containing magnesium aluminum silicate; and Example 5 - containing neither carbomer or magnesium aluminum silicate) were shown to separate within one week. It is concluded that carbomer is responsible for the physical stability of the emulsified aqueous tretinoin cream.

**Claims**

1. In a stable emulsified aqueous tretinoin cream formulation comprising a therapeutically active amount of tretinoin in a pharmaceutically acceptable aqueous carrier, the improvement which comprises including in the formulation as a stabilizer from about 0.01 to about 5 percent by weight of an acrylic acid polymer to prevent breaking of the emulsion.

2. The formulation of claim 1 wherein the acrylic acid polymer contains repeating units having the structure

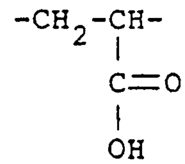

cross-linked with an allyl ether of pentaerythritol or an allyl ether of sucrose.

3. The formulation of claim 2 containing from about 0.005 to about 0.5 percent by weight of tretinoin; from about 1 to about 15 percent by weight of emulsifier; from about 5 to about 50 percent by weight of a hydrophobic material; from about 0.05 to about 3 percent by weight of preservative; from about 0.01 to about 1 percent by weight of anti-oxidants; and from about 0.01 to about 5 percent by weight of acrylic polymer.

4. The formulation of claim 3 wherein said hydrophobic material is selected from the group consisting of fatty acids having 12 to 20 carbon atoms, fatty acid esters in which the fatty acid portion has 6 to 20 carbon atoms, fatty alcohols having 12 to 20 carbon atoms, cocoa butter, mineral oil, petrolatum, bees wax, white wax, hydrocarbon wax, sperm wax, and silicone oils.

5. The formulation of claim 1 wherein the acrylic acid polymer contains repeating units of acrylic acid and a long chain alkyl methacrylate having the structure

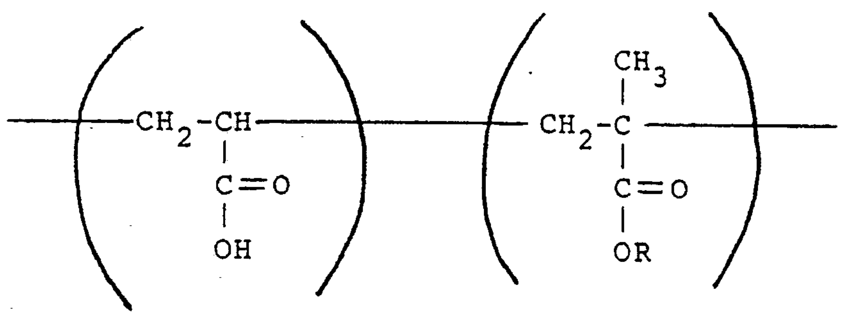

wherein R is an alkyl group or an alkenyl group containing from 10 to 30 carbon atoms cross-linked with an allyl ether of pentaerythritol or sucrose.

6. The formulation of claim 3 wherein the emulsifier is a non-ionic emulsifier.

7. The formulation of claim 4 wherein the fatty acid ester is selected from the group consisting of dibutyl adipate, diisopropyl adipate and other adipic acid esters.

8. The formulation of claim 3 wherein said acrylic acid polymer has an approximate molecular weight of from 450,000 to 4,000,000.

9. The formulation of claim 8 wherein said acrylic acid polymer has an approximate molecular weight of 3,000,000.

10. The formulation of claim 6 wherein the emulsifier is selected from the group consisting of polyoxyethylene-2-stearyl ether, polyethylene glycol-23-lauryl ether, polyoxyethylene-21-stearyl ether, polyoxyethylene-4-lauryl ether, polyethylene glycol-6-ceteryl ether, polyethylene glycol-25 ceteryl ether, sorbitan monostearate, polyoxyethylene-20-sorbitan monostearate and mixtures thereof.

11. The formulation of claim 3 wherein the preservatives are selected from the group consisting of diazolidinyl urea, benzyl alcohol, methyl paraben, propyl paraben, DMDM hydantoin, methyl isothiazolinone, methyl chloroisothiazolinone, and mixtures thereof.

12. The formulation of claim 3 wherein the anti-oxidants are selected from the group consisting of butylated hydroxyanisole, butylated hydroxytoluene, tocopherol, propyl gallate, and mixtures thereof.

13. The formulation of claim 1 containing from about 0.005 to about 0.5 percent by weight of tretinoin.

14. The formulation of claim 2 containing from about 0.005 to about 0.5 percent by weight of tretinoin.

15. The formulation of claim 3 containing from about 0.005 to about 0.5 percent by weight of tretinoin.

16. The formulation of claim 4 containing from about 0.005 to about 0.5 percent by weight of tretinoin.

17. A stable emulsified tretinoin cream formulation containing 0.1 parts by weight tretinoin, 6.0 parts by weight petrolatum, 1.0 parts by weight dimethyl polysiloxane, 1.6 parts by weight cetyl alcohol, 0.5 parts by weight polyoxyethylene-23-lauryl ether, 0.05 parts by weight butylated hydroxytoluene, 0.05 parts by weight butylated hydroxyanisole, 2.5 parts by weight polyoxyethylene-2-stearyl ether, 8.0 parts by weight cocoa butter, 10.0 parts by weight dibutyl adipate, 5.0 parts by weight glycerin, 0.25 parts by weight of an acrylic acid polymer which containing repeating units having the structure

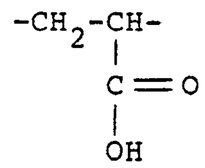

which is cross-linked with an allyl ether of sucrose and has an approximate molecular weight of 3,000,000, 0.30 parts by weight magnesium aluminum silicate, 0.05 parts by weight of a mixture of methylchloroisothiazolinone and methylisothiazolinone, 0.20 parts by weight diazolidinyl urea, 0.07 parts by weight sodium hydroxide, 0.05 parts by weight disodium ethylenediaminetetraacetic acid, and 64.28 parts by weight purified water.

18. A stable emulsified tretinoin cream formulation containing 0.05 parts by weight tretinoin, 6.0 parts by weight petrolatum, 1.0 parts by weight dimethyl polysiloxane, 1.6 parts by weight cetyl alcohol, 0.5 parts by weight polyoxyethylene-23-lauryl ether, 0.05 parts by weight butylated hydroxytoluene, 0.05 parts by weight butylated hydroxyanisole, 2.5 parts by weight polyoxyethylene-2-stearyl ether, 8.0 parts by weight cocoa butter, 10.0 parts by weight dibutyl adipate, 5.0 parts by weight glycerin, 0.25 parts by weight of an acrylic acid polymer which contains repeating units having the structure

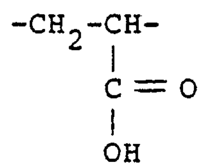

which is cross-linked with an allyl ether of sucrose and has an approximate molecular weight of 3,000,000, 0.30 parts by weight magnesium aluminum silicate, 0.05 parts by weight of a mixture of methylchloroisothiazolinone and methylisothiazolinone, 0.20 parts by weight diazolidinyl urea, 0.07 parts by weight sodium hydroxide, 0.05 parts by weight disodium ethylenediaminetetraacetic acid, and 64.33 parts by weight purified water.

19. A process for preparing the formulations of anyone of claims 1 to 18 which comprises admixing the components as defined in any of claims 1 to 18.

European Patent Office

# EUROPEAN SEARCH REPORT

Application Number

**EP 90 11 8828**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | GB-A-2 197 589 (L'OREAL)<br>* Page 6, line 5 - page 9, line 14; example 8 * | 1-19 | A 61 K 7/48 |
| A | FR-A-2 619 309 (L'OREAL)<br>* Page 15, line 13 - page 18, line 18; example 3 * | 1-19 | |
| A | EP-A-0 332 501 (LABORATORIES Dr. N.G. PAYOT)<br>* Whole document * | 1-19 | |
| A | US-A-4 214 000 (PAPA)<br>* Whole document * | 1-19 | |
| A | EP-A-0 274 104 (F. HOFFMANN-LA ROCHE & CO.)<br>* Whole document * | 1-19 | |

TECHNICAL FIELDS SEARCHED (Int. Cl.5)

A 61 K

The present search report has been drawn up for all claims

| Place of search | Date of completion of search | Examiner |
|---|---|---|
| The Hague | 10 January 91 | FISCHER J.P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document